# EUROPEAN PATENT SPECIFICATION

(11) **EP 2 381 864 B1**
(45) Date of publication and mention of the grant of the patent: **05.09.2012**
(21) Application number: 10776554.7
(22) Date of filing: 19.10.2010
(51) Int. Cl.: A61B 17/32, A61B 17/225, A61F 2/46, A61B 17/00

(54) **SHOCKWAVE APPARATUS HAVING A PNEUMATIC DRIVE**
STOSSWELLENVORRICHTUNG MIT PNEUMATISCHEM ANTRIEB
APPAREIL À ONDE DE CHOC AYANT UN ENTRAÎNEMENT PNEUMATIQUE

(30) Priority: 19.10.2009 DE 102009049924
(43) Date of publication of application: 02.11.2011
(73) Proprietor: STORZ MEDICAL AG, 8274 Tägerwilen (CH)
(72) Inventor: GLENZER, Thomas, CH-8280 Kreuzlingen (CH); FROESE, Klaus, 78465 Konstanz (DE); SCHULZ, Manfred, 8274 Tägerwilen/T (CH)
(74) Representative: Szynka, Dirk
(86) International application number: PCT/EP2010/006367
(87) International publication number: WO 2011/047827

(56) References cited:
- DE-U1- 9 218 254
- US-A- 3 930 505
- US-A- 5 160 336
- US-A1- 2008 142 093
- US-A1- 2008 289 398
- US-A1- 2009 221 940

## Description

### Field of the Invention

The present invention relates to a shock wave apparatus for treating the human or animal body by mechanical shockwaves.

### Background of the Invention

Such apparatuses are known as such, in particular in the area of lithotripsy. There, body concrements, in particular stones in the body tissue, are disintegrated by focused mechanical shockwaves. Besides the production by electrical discharges in water, also apparatuses have been developed producing the mechanical shockwaves by the collision of an accelerated projectile and an impact body and coupling said shockwaves into body tissue by means of said impact body. Such apparatuses have been used also in lithotripsy by a direct contact between the impact body or a probe connected to the impact body and the stone, and in other treatments of biological body substances. In particular, the treatment of muscle diseases and of diseases in the transition region between muscles and bones are to be named.

A pneumatic implementation of the drive of the projectile is principally known, for example from DE 20 2004 011 323 U1. A compressor compresses for example air as a pressure gas to be used for accelerating the projectile in a guiding tube. The compressor has a motor, usually an electric motor. The compressor and its motor are steadily operated during use in the prior art. Patent document US 5160336 shows an apparatus according to the preamble of claim 1.

### Brief Summary of the Invention

The present invention has the object to improve a shockwave apparatus of the above described type with regard to its properties of use in terms of the compressor operation.

Here to, the invention is directed to a shockwave apparatus for treating a human or animal body having a pneumatic drive for producing a shockwave to be coupled into said body and a compressor of said pneumatic drive for producing pressure gas, said compressor comprising a compressor motor, characterized in that the rotation rate of said compressor motor is adjustable for setting a gas pressure for producing said shockwave.

Preferred embodiments of the apparatus according to the invention and its use are defined in the dependent claims. The features comprised therein and the disclosure of the following description are to be understood in view of both categories of the invention without any explicit differentiation there between herein.

The basic idea of the invention is to be able to adjust the motor rotation rate of the compressor motor, namely by the user and not only in the production. The shockwave apparatus thus comprisses a device for adjusting this rotation rate or for manipulating the rotation rate. By means of the rotation rate adjustment, the pressure producing the shockwave shall be manipulated and thus the intensity of the individual impact process. The device can also be designated as a device for adjusting the pressure, the pulse energy or the like.

In contrast thereto, steadily running compressor motors are used in the prior art and the pressures are predetermined by adjusting pressure reducing valves at the output side of the compressors. This has the advantage to be able to work with simple motor controllers. However, the inventors have found substantial disadvantages.

For example, a pressure reducing valve principally needs a minimal pressure difference. The conventional compressor thus has to produce a higher pressure than needed even in case of a maximum adjustment of the pressure. This is the reason for noise, power consumption and, in the design of the apparatus, a somewhat bigger power class of the compressor.

This applies even more to the very frequent case that a smaller pressure than the maximal possible pressure is desired. In such cases, a correspondingly larger difference pressure is discharged to the environment so that the compressor causes noise and consumes energy at an unnecessary level. Further, the pressure reducing valve or a pressure regulating means that has been necessary in the prior art can be omitted.

A use of a feedback control circuit for a feedback control of the pressure by the motor rotation rate is preferred, in particular including a PID feedback control circuit (proportional, integral, differential feedback control circuit). Therein, for example the output pressure of the compressor is detected as a feedback value and is feedback controlled by manipulating the motor rotation rate as a manipulated variable to a target value.

A practical and economic implementation of the feedback control circuit provides a feedback control implemented as software. The software can run for example on a micro-controller or more generally spoken, on a programmable integrated circuit. Here, but also in a hardware implementation of the feedback control circuit, a so called one-quadrant feedback control is preferred, i.e. a feedback control operating with positive feedback deviations only. In the present case this means that the one-quadrant feedback control is in control operation only for smaller target values than the feedback value and that the motor is switched off in the opposite case.

Further, the shockwave apparatus according to the invention preferably comprises a motor run-up current limiter, preferably implemented in software, and more preferably running on the same computer or programmable device. This run-up current limiter can be a PI feedback control circuit (proportional integral feedback control circuit), in particular.

The motor output portion feeding the compressor motor, for example a half bridge, is preferably driven by a pulse width modulation method, i.e. by a PWM control. In this context, a limitation of the PWM pulses both with regard to the positive and to the negative amplitudes is preferred as explained along the exemplary embodiment.

A particularly simple and practical implementation of the compressor is an air-cooled compressor. Although such compressors are louder than oil-cooled compressors, they are substantially lighter and thus easier to be transported. In this invention, the increased noise levels can be reduced and thus can be kept in an acceptable range. Further, a part of the elements of the shockwave apparatus is preferably implemented in a hand device and is thus adapted to be held by the hand of the user, said hand device being connected to a stationary base station by a supply line, in particular a pneumatic line. Naturally, the base station can be displaceable or can be carried along, however, it is stationary during operation in a regular case. In particular the compressor is located here.

The prior art cited in the beginning uses a projectile to be accelerated by the pressure gas. Such an embodiment is preferred in this invention as well but is not mandatory.

Preferably, the projectile can be moved along a guiding tube and produces the shockwave by an impact at an end of the guiding tube. The guiding tube thus serves as an acceleration path.

Further, the invention is directed to shockwave apparatus comprising impact bodies or transfer elements, i.e. such solid bodies that are arranged at a distal end or impact-side end of the guiding tube and serve for an impact of the projectile. Such impact bodies are placed onto the body to be treated directly or indirectly and can couple energy into the body by an elastic wave and by an macroscopic movement of the impact body. In this regard, reference is made to the utility model already cited.

Principally, projectiles can also be used without a linear guiding tube. For illustration, reference is made to DE 10 2006 057 268 in which projectiles running along a circular path are described.

Further, the projectile can be omitted completely and a pneumatic pressure gas front can hit the impact body directly. For illustration, reference is made to DE 20 2007 007 921.

Finally, it is possible to use a projectile but no impact body. The projectile can collide with the body surface to be treated directly at the end of its movement path, as an example.

As a last remark, in principle modifications are feasible in which a pressure gas front hits the body surface directly although such embodiments are not preferred. Preferred applications relate to soft tissue, in particular muscles and muscle-bases, acupuncture, enthesis treatment, trigger point therapy.

Hereunder, the invention will be explained in more detail along an exemplary embodiment, wherein the individual features thereof can also be important in other combinations and relate, as already mentioned, to all categories of the invention, implicitly.

### Brief Description of the Drawings

- Figure 1: shows an apparatus according to the invention in longitudinal section and having a schematically illustrated pneumatic drive.
- Figure 2: shows, as a block circuit diagram, parts of a control 44 of figure 1 comprising a feedback control circuit for pressure feedback control.

### Detailed Description of the Invention

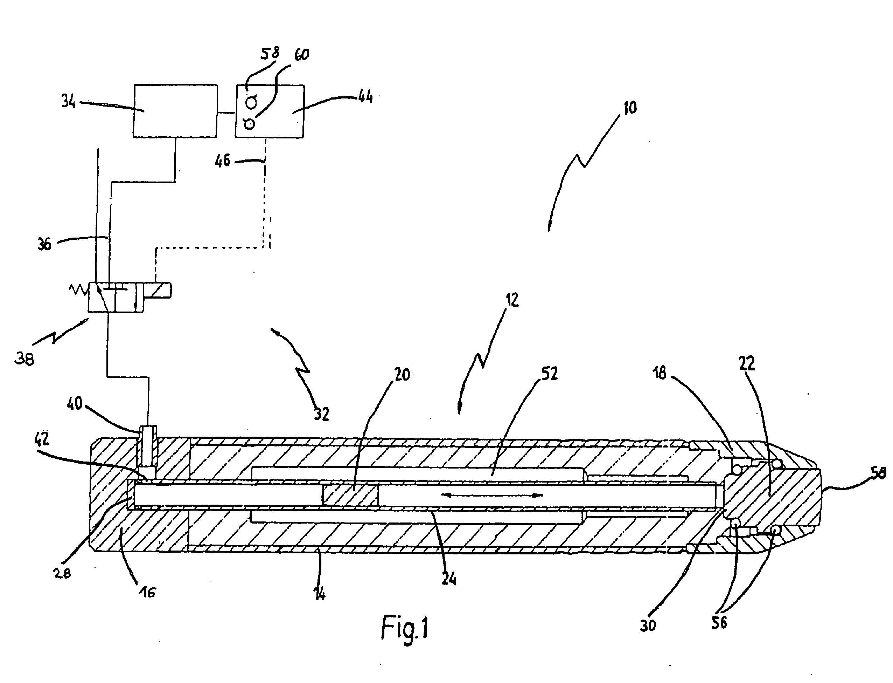

Figure 1 shows a medical apparatus for treating the human body by mechanical shockwaves, being designated by 10, in this case adapted for a soft tissue treatment in the context of a pain treatment. The apparatus consists of a hand-piece 12 and a pneumatic pressure gas supply device 32 to be explained below in more detail. A medical doctor responsible for treatment, as an example, can grip the hand-piece 12 and position the right end in Figure 1 onto a suitable skin portion wherein the hand-piece 12 is approximately orthogonal to the skin.

A casing 14 has a proximal terminal cap 16 and a distal terminal cap 18 being removable respectively. A guiding tube 24 is held in the casing and is arranged axially and concentrically. A projectile 20 is guided in the guiding tube, the movement path of which projectile along the interior of the guiding tube 24 being limited on the right side by an impact body 22, namely by its proximal side 30. This constitutes a distal abutment stop for the projectile 20 wherein the proximal abutment stop of the projectile 20 is designated by 28 and is a simple closure of the guiding tube 24. This closure is magnetic so that the projectile 20 can be fixed along by certain holding force. Typically, the length of the guiding tube 24 is about 5 cm - 20 cm.

The pneumatic drive 32 implements the pressure gas supply device and comprises a common pneumatic compressor 34, wherein the compressor 34 has a typical operation range up to about 10 bar wherein only about up to 5 bar or up to 8 bar are needed. A pressure gas terminal 40 of the hand-piece 12 is supplied via a pressure conduit 36 and a switching valve 38, which terminal 40 communicates with the guiding tube 24 via the opening 42 therein. The switching valve 38 can be a magnetic valve. A control 44 is connected thereto via a control line 46 being illustrated by a hatched line. The control 44 can be implemented as a structural unit with the compressor 34 and thus constitute a basic device for supplying the hand-piece 12 wherein the switching valve 38 is advantageously arranged at the latter. Correspondingly, the control 44 and the compressor 34 in Figure 1 are connected by a line. The basic device and the hand-piece 12 are then connected via a pneumatic conduit 36 and the control line 46 combined in a supply line.

Two adjustment buttons 58 and 60 are provided on the control 44 whereby the supply pressure provided by the conduit 36 and the operation frequency of the switching valve 38 can be set. The adjustment button 58 serves for adjusting the pressure output of the compressor 34, namely by a target value setting for a PID feedback control circuit in the control 44 being explained in more detail hereunder. Hereto the control 44 is connected to the compressor 34 by a line shown.

Further, the control 44 is adapted to control the switching valve 38 with a frequency set at the adjustment button 60 in a range of 0 Hz - 50 Hz.

Before a more detailed explanation of the control 44, the working method of the apparatus in figure 1 will be summarized:

Starting from a non-operating condition of the apparatus 10, i.e. at the start of operation, the closed switching valve 38 is opened by the control 44. The condition shown in Figure 1 in which the guiding tube 24 is connected to the exterior atmosphere is then changed into a condition shown by the right square of the valve symbol where-in the supply pressure is applied to the guiding tube 24 via the terminal 40. Therein, the projectile 20 is in its original position, first, designated by 48 in Figure 1. The rising pressure accelerates the projectile 20 towards the impact body and is decreased even before the collision by a back-switching of the switching valve 38 and thus by a ventilation of the volume "behind" the projectile 20 in the guiding tube 24, however. The projectile 20 hits the impact body 22 directly, the distal (somewhat convex) terminal surface 58 of which is positioned on the skin of the patient and transfers a mechanical shockwave into the body. Therein, the impact body 22 is subjected to an axial travel due to its elastic suspension in the two elastomer O-rings 56. Directly after the collision, the projectile 20 is moved backwards. This is assisted by a counterpressure chamber 52 being connected to the guiding tube 24, namely its distal end short before the proximal side 30 of the impact body 22, in a manner not shown in detail here. In this counterpressure chamber, a counterpressure returning the projectile 20 after the collision up to the proximal stop, namely the magnetic terminal piece 28, results from the air shift due to the movement of the projectile 20. After a certain time, the switching valve 38 is switched again so that a new trigger process results. This certain time and the on-time of the switching valve 38 make up the inverse value of the frequency set, together. Typical collision velocities of the projectile are in the range of 5 m/s - 60 m/s, in particular of 5m/s to 30m/s.

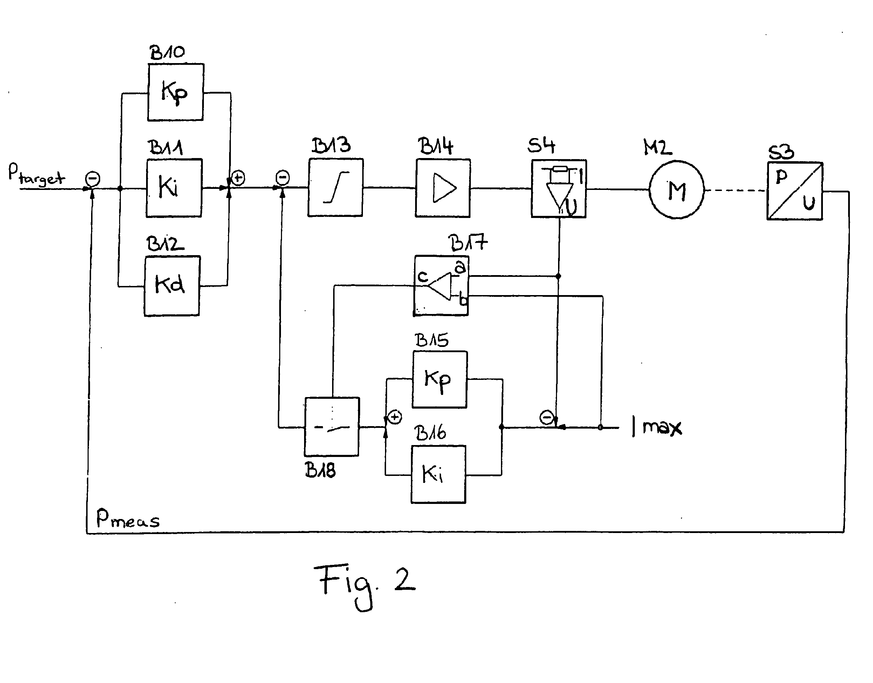

Figure 2 shows a block circuit diagram of the working structure of the control 44. An electric motor M2 of the compressor 34 of figure 1 is labeled by M and shown as a circle in the right portion. A pressure sensor S3 is integrated at the compressor output and detects the output pressure P_{MEAS} directly at the compressor casing for simplicity and to avoid substantial conduit lengths. This pressure sensor S3 supplies a voltage value U proportional to the pressure P_{IST} via a line shown to a differentiating element on the left side of figure 2 at the input of a PID feedback control circuit. There, the feedback value P_{IST} is subtracted from the target value P_{TARGET} and the difference thereof is supplied to a PID element known as such. Therein, the target value P_{TARGET} originates from an adjustment of adjustment button 58 on the control 44. Thus, the line between the sensor S3 and the differentiating element is a part of the line between the compressor 34 and the control 44 in figure 1.

The PID element consists of a proportional element having the symbol Kₚ and the reference B10, an integrating element Kᵢ having the reference B11 and a differentiating element K_{d} having the reference B12. They are connected in parallel as usual and process a control difference in an individual and adjustable manner, respectively. Their outputs are added and transformed in a manner not shown for manipulating a PWM control signal of a PWM control known as such of motor output portion. The PWM control signal is limited by a limiter B13 to a range of operation of the control between a maximum and a minimum value and supplied to the motor output portion B14, namely a PWM controlled transistor half bridge. The output current of the motor output portion B14 passes a current detector element S4 for than driving the already mentioned motor M2 of the compressor 34.

In the present embodiment, the motor M2 is a direct current motor. Further, the compressor 34 works along the membrane principle. Thus, the compressor produces pressure in both rotational senses of the motor. Consequently, the PID feedback control can be limited to a quadrant, i.e. to values of only one polarity, because with opposite polarity, only a symmetric situation would result. Thus, the PWM controlled transistor half bridge can be constructed particularly simple. The pulse duty factor of the pulse with modulated control corresponds to the motor voltage therein.

Naturally, also an alternating current motor and a converter could be used wherein the motor would be controlled by the frequence instead of the voltage. Similarly, synchronous motors and asynchronous motors are contemplated. Respective alternative embodiments of the invention are clear to the skilled reader and detailed explanation can be omitted here.

The current feedback value detected in the current detection S4 element is used for a PI feedback control circuit for current limitation. Hereto, it is fed into a differentiating element and subtracted from a predetermined maximum value Iₘₐₓ therein. This maximum value Iₘₐₓ can be set during production and can be adapted to the respective motor used and its current supply. The difference is an input of a PI feedback control element known as such having a proportional element B15 and an integrating element B16, the outputs of which respond individually and adjustably to the feedback difference. They are added and fed into the differentiating element as already mentioned at the input of the limiter B13. However, this is done only if the current feedback value of the current detecting element S4 exceeds a certain threshold. Hereto, the latter is used as an input a of a comparator B17, the reference input b of which receives the current maximum value Iₘₐₓ already mentioned. In case of a negative difference, namely if the current detected is larger than the maximum allowed current, a switch B18 at the output of the adding element at the rear side of the PI-feedback control element B15, B16 is closed in order to guarantee a feedback control action of the PI-feedback control circuit and to limit the current to allowed values. In the other case, the PI-feedback control circuit remains open and without effect.

In the manner described right now, disadvantageously large run-up currents of the compressor motor M2 can be avoided. Such run-up currents appear at a start from a stillstand condition, in particular if the compressor motor works against a load. The latter condition can be a desired one, namely if the volume at the downstream side the compressor motor remains under pressure in a switch-off condition of the compressor motor in order to supply the necessary operating pressure after a restart as quickly as possible. In particular in such situations, the run-up current limitation explained is advantageous.

The contents of the diagram of the figure 2 is preferably implemented in software, naturally with the exception of the compressor motor M2, the pressure censor S3 and the motor output portion B14, namely as a program of an 8-bit microcontroller such as the type microchip PIC16. Here, a sampling rate of 5 to 20 Hz can be used wherein the algorithms of the PID-element can be operated with 300 to 400 µs and the algorithms of the PI-element with 50 µs. This concept is very flexible and easy to change as a program and is easily adapted for various designs of the same basic device model, individually. Moreover, it is very economic.

A conventional shockwave apparatus can easily be equipped according to the invention by removing a pressure reducing valve, mounting setting elements (potentiometers) for a target value setting and integrating the contents of figure 2 into the operation software. For the PWM-control, a respectively adapted motor output portion has to be provided.

Further, a display for the pressure target value can be integrated such as by a program change of the operating software controlling a display present anyway or by adding a display element.

Some quantitative examples can illustrate the advantages of the invention:

A typical compressor has a characteristic curve in the sense of volume rates (transport powers) achievable at certain pressures, naturally enabling larger volume rates at lower pressures and smaller volume rates at higher pressures. The prior art operates the compressor with a fixed rotation rate of the compressor motor and along this characteristic curve in this manner. Actually, the characteristic curve is only an upper limit of a working area, the lower working points not being used in the prior art. If for example at a certain desired working frequency of the switching valve, a certain transport power of for example 4 l/min is necessary and the compressor produces 6 bar output pressure at this transport power, maybe only 3 bar operating pressure are desired. In this case, the pressure reducing valve discharges the pressure difference to the environment or, to be somewhat more correct, discharges a pressure gas volume, namely for example 3.5 l/min. These 3.5 l/min pressure gas are the difference between 4 l/min as required and 7.5 l/min actually possible at a pressure of 3 bar. According to the invention, the compressor would be operated by somewhat more than half of the rotation rate in this situation and no pressure reducing valve would be used. Thus, the noise and the energy consumption of the compressor are substantially reduced.

## Claims

1. A shockwave apparatus (10) for treating a human or animal body having a pneumatic drive (32) for producing a shockwave to be coupled into said body and
a compressor (34) of said pneumatic drive (32) for producing pressure gas, said compressor (34) comprising a compressor motor,
**characterized in that** said shockwave apparatus (10) comprises a device (44) for adjusting the rotation rate of said compressor motor for setting an accelerating gas pressure.

2. The shockwave apparatus (10) of claim 1 wherein an output of said compressor is connected to a guiding tube without a pressure reducing valve.

3. The shockwave apparatus (10) of claim 1 or 2 having a feedback control circuit (B10 - B14, S3) for feedback controlling said gas pressure by said rotation rate of said compressor motor as a manipulated value.

4. The shockwave apparatus (10) of claim 3 wherein said feedback control circuit (B10 - B14, S3) comprises a PID-feedback control circuit (B10 - B12).

5. The shockwave apparatus (10) of claim 3 or 4 wherein said feedback control circuit (B10 - B14, S3) is implemented as software.

6. The shockwave apparatus (10) of claim 5 wherein said software is loaded in a micro-controller.

7. The shockwave apparatus (10) of one of claims 3 to 6 wherein said feedback control circuit (B10 - B14, S3) is a one-quadrant feedback control circuit.

8. The shockwave apparatus (10) of one of the preceding claims having a run-up current limiter (S4, B15 - B18) for limiting run-up currents of said compressor motor.

9. The shockwave apparatus (10) of claim 8 wherein said run-up current limiter comprises a PI feedback control circuit (B15, B16).

10. The shockwave apparatus (10) of one of the preceding claims having a PWM control (B13) of a motor output portion (B14) for operating said compressor motor.

11. The shockwave apparatus (10) of one of the preceding claims wherein said compressor (34) is an air-cooled compressor.

12. The shockwave apparatus (10) of one of the preceding claims having a hand device (12) and a base station (34, 44), said base station (34, 44) comprising said compressor (34) and being connected to said hand device (12) by a conduit (36, 46).

13. The shockwave apparatus (10) of one of the preceding claims having a striking element (20) moveable by said pneumatic drive (32) and adapted for a collision for producing said shockwaves.

14. The shockwave apparatus (10) of claim 13 having a guiding tube (24) in which said striking element (20) is moveable in order to produce said shockwaves by said collision at the end of a movement through said guiding tube (24).

15. The shockwave apparatus (10) of one of the preceding claims having an impact body (22) for producing said shockwaves as a result of a pressure pulse of said pneumatic drive (32).

## Patentansprüche

1. Druckwellengerät (10) zur Behandlung des menschlichen oder tierischen Körpers mit
einem pneumatischen Antrieb (32) zur Erzeugung einer Druckwelle zur Einkopplung in den Körper und
einem Kompressor (34) des pneumatischen Antriebs (32) zur Erzeugung von Druckgas, welcher Kompressor (34) einen Kompressormotor aufweist,
**dadurch gekennzeichnet, dass** das Druckwellengerät (10) eine Einrichtung (44) zur Einstellung der Drehzahl des Kompressormotors zur Einstellung des beschleunigenden Gasdrucksaufweist.

2. Druckwellengerät (10) nach Anspruch 1, bei dem ein Ausgang des Kompressors ohne Druckminderer an das Führungsrohr angeschlossen ist.

3. Druckwellengerät (10) nach Anspruch 1 oder 2 mit einem Regelkreis (B10-B14, S3) zur Regelung des Gasdrucks mit der Drehzahl des Kompressormotors als Stellgröße.

4. Druckwellengerät (10) nach Anspruch 3, bei dem der Regelkreis (B10-B14, S3) einen PID-Regler (B10-B12) aufweist.

5. Druckwellengerät (10) nach Anspruch 3 oder 4, bei dem der Regelkreis (B10-B14, S3) als Software realisiert ist.

6. Druckwellengerät (10) nach Anspruch 5, bei dem die Software in einen Mikrocontroller geladen ist.

7. Druckwellengerät (10) nach einem der Ansprüche 3 bis 6, bei dem der Regelkreis (B10-B14, S3) ein Einquadrantenregler ist.

8. Druckwellengerät (10) nach einem der vorstehenden Ansprüche mit einem Anlaufstrombegrenzer (S4, B15-B18) zur Begrenzung von Anlaufströmen des Kompressormotors.

9. Druckwellengerät (10) nach Anspruch 8, bei dem der Anlaufstrombegrenzer einen PI-Regler (B15, B16) aufweist.

10. Druckwellengerät (10) nach einem der vorstehenden Ansprüche mit einer PWM-Steuerung (B13) einer Motorendstufe (B14) zum Betrieb des Kompressormotors.

11. Druckwellengerät (10) nach einem der vorstehenden Ansprüche, bei dem der Kompressor (34) ein luftgekühlter Kompressor ist.

12. Druckwellengerät (10) nach einem der vorstehenden Ansprüche mit einem Handgerät (12) und einer Basisstation (34, 44), die den Kompressor (34) aufweist und mit dem Handgerät (12) über eine Leitung (36, 46) verbunden ist.

13. Druckwellengerät (10) nach einem der vorstehenden Ansprüche mit einem Schlagteil (20), das durch den pneumatischen Antrieb (32) bewegbar und zu einem Aufprall zur Erzeugung der Druckwelle ausgelegt ist.

14. Druckwellengerät (10) nach Anspruch 13 mit einem Führungsrohr (24), in dem das Schlagteil (20) bewegbar ist, um am Ende einer Bewegung durch das Führungsrohr (24) die Druckwelle durch den Aufprall zu erzeugen.

15. Druckwellengerät (10) nach einem der vorstehenden Ansprüche mit einem Prallkörper (22) zur Erzeugung der Druckwelle infolge eines Druckpulses des pneumatischen Antriebes (32).

## Revendications

1. Appareil à onde de choc (10) destiné au traitement d'un corps humain ou animal, présentant:
un entraînement pneumatique (32) destiné à produire une onde de choc devant être couplée dans ledit corps, et
un compresseur (34) dudit entraînement pneumatique (32) destiné à produire un gaz de compression, ledit compresseur (34) présentant un moteur de compresseur,
**caractérisé en ce que** ledit appareil à onde de choc (10) comprend un dispositif (44) destiné à régler la vitesse de rotation dudit moteur de compresseur afin d'établir une pression de gaz d'accélération.

2. Appareil à onde de choc (10) selon la revendication 1, dans lequel un orifice de sortie du compresseur est raccordé à un tube de guidage sans réducteur de pression.

3. Appareil à onde de choc (10) selon la revendication 1 ou 2, présentant un circuit de commande à rétroaction (B10 - B14, S3) servant à commander par rétroaction ladite pression de gaz en utilisant ladite vitesse de rotation dudit moteur de compresseur comme valeur de réglage.

4. Appareil à onde de choc (10) selon la revendication 3, dans lequel ledit circuit de commande à rétroaction (B10 - B14, S3) comprend un circuit de commande à rétroaction du type PID (B10 - B12).

5. Appareil à onde de choc (10) selon la revendication 3 ou 4, dans lequel ledit circuit de commande à rétroaction (B10 - B14, S3) est mis en oeuvre sous la forme d'un logiciel.

6. Appareil à onde de choc (10) selon la revendication 5, dans lequel ledit logiciel est chargé dans un microcontrôleur.

7. Appareil à onde de choc (10) selon l'une des revendications 3 à 6, dans lequel ledit circuit de commande à rétroaction (B10 - B14, S3) est un circuit de commande à rétroaction à quadrant unique.

8. Appareil à onde de choc (10) selon l'une des revendications précédentes, présentant un limiteur de courant de démarrage (S4, B15 - B18) destiné à limiter les courants de démarrage dudit moteur de compresseur.

9. Appareil à onde de choc (10) selon la revendication 8, dans lequel ledit limiteur de courant de démarrage comprend un circuit de commande à rétroaction de type proportionnel-intégral (B15, B16).

10. Appareil à onde de choc (10) selon l'une des revendications précédentes, présentant une commande PWM (B13) d'un étage de sortie de moteur (B14) pour exploiter ledit moteur de compresseur.

11. Appareil à onde de choc (10) selon l'une des revendications précédentes, dans lequel ledit compresseur (34) est un compresseur refroidi par air.

12. Appareil à onde de choc (10) selon l'une des revendications précédente, présentant un appareil manuel (12) et un poste de base (34, 44), ledit poste de base (34, 44) comprenant ledit compresseur (34) et étant raccordé audit appareil manuel (12) par une conduite (36, 46).

13. Appareil à onde de choc (10) selon l'une des revendications précédentes, présentant un élément de frappe (20) susceptible d'être déplacé sous l'effet dudit entraînement pneumatique (32) et adapté à une collision pour produire lesdites ondes de choc.

14. Appareil à onde de choc (10) selon la revendication 13, présentant un tube de guidage (24) dans lequel ledit élément de frappe (20) peut être déplacé pour produire lesdites ondes de choc sous l'effet de ladite collision à la fin d'un déplacement dans ledit tube de guidage (24).

15. Appareil à onde de choc (10) selon l'une des revendications précédentes, présentant un corps d'impact (22) servant à produire lesdites ondes de choc en conséquence d'une impulsion de compression dudit entraînement pneumatique (32).
